# FASCICULE DE BREVET EUROPEEN

(11) **EP 1 800 656 B1**
(45) Date de publication et mention de la délivrance du brevet: **01.06.2011**
(21) Numéro de dépôt: 06291960.0
(22) Date de dépôt: 18.12.2006
(51) Int. Cl.: A61K 8/49, A61Q 17/00

(54) **Utilisation d'un ester de sorbitan oxyalkyléné en tant qu'agent apaisant du cuir chevelu**
Verwendung eines Oxyalkylensorbitanesters als beruhigendes Mittel der Kopfhaut
Use of an polyoxyalkylenesorbitan ester as a soothing agent for scalp

(30) Priorité: 22.12.2005 FR 0513192
(43) Date de publication de la demande: 27.06.2007
(73) Titulaire: L'Oréal, 75008 Paris (FR)
(72) Inventeur: Decoster, Sandrine, 95210 Saint-Gratien (FR); Mezure, Patricia, 78380 Bougival (FR)
(74) Mandataire: Dossmann, Gérard

(56) Documents cités:
- DE-A1- 19 537 509
- FR-A- 2 303 076
- FR-A- 2 804 020
- US-A- 5 756 437
- US-B1- 6 589 516
- TURKOGLU M ET AL: "EVALUATION OF IRRITATION POTENTIAL OF SURFACTANT MIXTURES" INTERNATIONAL JOURNAL OF COSMETIC SCIENCE, KLUWER ACADEMIC PUBLISHERS, DORDRECHT, NL, vol. 21, no. 6, décembre 1999 (1999-12), pages 371-382, XP001002684 ISSN: 0142-5463
- ANONYMOUS: "Final report on the safety assessment of PEG-20 Sorbitan Cocoate;PEG-40 Sorbitan Diisostearate; PEG-2, -5, and -20 Sorbitan Isostearate; PEG-40 and -75 Sorbitan Lanolate; PEG-10, -40, -44, -75, and -80 Sorbitan Laurate; PEG-3, and -6 Sorbitan Oleate; PEG-80 Sorbitan Palmitate; PEG-40 Sorbitan Periso" BIOSIS, 2000, XP002233306
- WILHELM K.P. ET AL: "Quantitative assessment of primary skin irritants in vitro in a citotoxicity model: comparison with in vivo human irritation tests"[Online] vol. 145, no. 5, novembre 2001 (2001-11), pages 709-715, XP002400613 Extrait de l'Internet: URL:http://www.blackwell-synergy.com/doi/f ull/10.1046/j.1365-2133.2001.04497.x> [extrait le 2006-09-26]

## Description

La présente invention est relative à l'utilisation d'un composé particulier de la famille des esters de sorbitan oxyalkylénés, en tant qu'agent apaisant dans une composition capillaire, destinée à apaiser le cuir chevelu.

De nombreux individus possèdent un cuir chevelu naturellement sensible, qui donne lieu à des phénomènes d'irritations et de démangeaisons. Par ailleurs, les cuirs chevelus, même peu sensibles, peuvent être irrités par des agressions extérieures telles que les intempéries, la transpiration, les lavages fréquents des cheveux, l'utilisation de produits cosmétiques capillaires agressifs tels que par exemple des produits de teinture capillaire ou de permanente, et peuvent alors présenter des zones de démangeaison.

Pour remédier à cet inconfort, il est connu d'appliquer sur le cuir chevelu des compositions non rincées, généralement hydroalcooliques.

Par ailleurs, l'utilisation d'esters de sorbitan oxyalkylénés en tant que tensioactifs dans des compositions de traitement cosmétique des cheveux a été décrite dans l'art antérieur.

Ainsi, la demande de brevet FR 2 804 020 décrit des compositions de lavage douces, notamment des shampooings, comprenant au moins un tensioactif détergent et au moins un ester d'acide gras et de sorbitan oxyéthyléné ayant un nombre de moles d'oxyéthylène inférieur ou égal à 10. L'utilisation d'un tel ester de sorbitan dans le système émulsifiant permet de diminuer le potentiel irritant pour les yeux par rapport à un shampooing classique contenant uniquement des tensioactifs détergents.

Le brevet EP 1 430 867 décrit un procédé amélioré pour la préparation de nanoémulsions cationiques de type huile-dans-eau, destinées au traitement cosmétique des matières kératiniques. Ces nanoémulsions comprennent au moins un tensioactif cationique, et au moins un tensioactif non ionique. Les tensioactifs non ioniques préférés pour la préparation de ces nanoémulsions incluent, entre autres, les mono-oléates de sorbitan à 4, à 5 ou à 20 OE.

La demande de brevet WO 2004/089318 décrit des compositions de shampooings présentant une action détergente et antipelliculaire améliorée. Ces compositions contiennent :
(a) de 5 à 50 % en poids d'un tensio-actif détergent,
(b) de 0,1 à 4 % en poids d'un agent anti-pelliculaire,
(c) de 0,1 à 50% en poids d'un dérivé polyoxyéthyléné d'un ester d'acide gras et de sorbitan, et
(d) au moins 20% en poids d'eau.

Le composé (c) est dérivé d'un ester de sorbitan et d'un acide gras en C14 à C18 tel que les acides palmitique, stéarique, oléique. Il est utilisé en tant que tensioactif.

La demande de brevet EP 0 490 053 décrit une composition de soin capillaire sous forme d'une microémulsion comprenant :
(a) de 5 à 20% en poids d'un tensioactif non ionique de HLB comprise entre 5 et 12 ou d'un mélange de tels tensioactifs, la HLB du mélange étant comprise entre 6 et 10,
(b) de 5 à 20% en poids d'au moins une huile,
(c) de 0,5 à 10% en poids d'au moins un tensioactif cationique, et
(d) de 50 à 89,5% en poids d'eau,
cette composition ne comprenant pas de tensioactif non ionique de HLB supérieure à 12. Comme tensioactifs non ioniques utilisables dans ces microémulsions, sont cités, entre autres, les esters d'acides gras en C₁₂ à C₁₈ et de sorbitan oxyéthylénés ayant de 1 à 6 OE.

Ainsi, les esters de sorbitan oxyalkylénés ont été employés, dans les compositions capillaires décrites dans l'art antérieur, avec une fonction de tensioactifs ou agents émulsifiants.

La Demanderesse a maintenant découvert de manière surprenante que l'utilisation, dans un milieu aqueux ou hydroalcoolique, de certains esters d'acides gras et de sorbitan oxyalkylénés, permettait d'apaiser de manière très efficace le cuir chevelu, c'est-à-dire de prévenir l'apparition des réactions d'inconfort, de diminuer l'intensité de ces réactions, voire de les faire disparaître.

En particulier, ces esters permettent de préparer des lotions ou d'autres produits de soin capillaire particulièrement efficaces contre les phénomènes d'inconfort du cuir chevelu, tels que les irritations, les démangeaisons, les picotements, les rougeurs.

La présente invention a donc pour objet un ester d'acide gras et de sorbitan oxyalkyléné comprenant de 1 à 20 unités oxyalkylène pour son utilisation en tant qu'agent apaisant dans une composition cosmétique aqueuse ou hydroalcoolique destinée au soin du cuir chevelu.

La présente invention concerne également un ester d'acide gras et de sorbitan oxyalkyléné comprenant de 1 à 20 unités oxyalkylène pour son utilisation, pour diminuer l'intensité des réactions d'inconfort du cuir chevelu, voire de les faire disparaître.

L'invention concerne en outre un procédé cosmétique de prévention des réactions d'inconfort du cuir chevelu, caractérisé en ce qu'il comprend l'application sur le cuir chevelu d'une composition cosmétique comprenant, en tant qu'agent apaisant, au moins un ester d'acide gras et de sorbitan oxyalkyléné comprenant de 1 à 20 unités oxyalkylène.

Selon un mode réalisation avantageux de l'invention, un tel procédé de prévention est employé en prétraitement cosmétique, afin de prévenir l'apparition des réactions d'inconfort du cuir chevelu susceptibles de survenir lors d'un traitement cosmétique capillaire ultérieur tel que par exemple une coloration, une décoloration, une permanente, un défrisage.

Ainsi, l'invention concerne également un procédé de traitement cosmétique capillaire comprenant au moins deux étapes :
- une première étape de prétraitement, destinée à prévenir l'apparition des réactions d'inconfort du cuir chevelu, et qui comprend l'application sur le cuir chevelu d'une composition cosmétique comprenant au moins un ester d'acide gras et de sorbitan oxyalkyléné comprenant de 1 à 20 unités oxyalkylène, et
- une deuxième étape, postérieure à ladite première étape, de traitement cosmétique capillaire choisi parmi une coloration, une décoloration, une permanente et un défrisage.

Au sens de la présente invention, on entend par apaiser le cuir chevelu le fait tant de prévenir l'apparition sur le cuir chevelu d'au moins une réaction d'inconfort que le fait de diminuer l'intensité d'une telle réaction, voire de la supprimer, lorsque celle-ci est déjà présente sur le cuir chevelu.

Par réaction d'inconfort, on désigne les réactions inesthétiques et/ou inconfortables du cuir chevelu, telles que par exemple l'échauffement, les démangeaisons, les picotements, les rougeurs.

D'autres objets et caractéristiques, aspects et avantages de l'invention apparaîtront plus clairement à la lecture de la description et des exemples qui suivent.

Dans le présent exposé, et de manière bien connue en soi, on désigne par « composé à X OE » un composé oxyéthyléné comprenant X unités oxyéthylène par molécule.

On désigne de plus par « composé en CY » un composé comprenant Y atomes de carbone.

Les composés utilisables dans la présente invention sont des esters d'acide gras et de sorbitan oxyalkylénés comprenant de 1 à 20 unités oxyalkylène.

Les unités oxyalkylène peuvent être notamment des unités oxyéthylène, ou oxypropylène. De préférence, l'ester d'acide gras et de sorbitan est oxyéthyléné.

De préférence, sont employés des dérivés oxyéthylénés des mono et des polyesters d'acides gras en C₈₋₃₀ et du sorbitan, ayant de 1 à 20 motifs d'oxyde d'éthylène. On utilise de préférence les dérivés oxyéthylénés des mono et des polyesters d'acides gras en C₁₂₋₂₄ et du sorbitan, ayant de 4 à 20 motifs d'oxyde d'éthylène.

De tels composés sont également connus sous le nom de polysorbates. Ils sont entre autres commercialisés sous la dénomination TWEEN par la société UNIQEMA. Citons par exemple : le mono-laurate de sorbitan oxyéthylène à 4 OE, commercialisé sous la dénomination TWEEN 21, le mono-laurate de sorbitan oxyéthylène à 20 OE, commercialisé sous la dénomination TWEEN 20, le mono-palmitate de sorbitan oxyéthylène à 20 OE commercialisé sous la dénomination TWEEN 40, le mono-stéarate de sorbitan oxyéthylène à 20 OE commercialisé sous la dénomination TWEEN 60, le mono-stéarate de sorbitan oxyéthylène à 4 OE commercialisé sous la dénomination TWEEN 61, le tri-stéarate de sorbitan oxyéthylène à 20 OE commercialisé sous la dénomination TWEEN 65, le mono-oléate de sorbitan oxyéthylène à 20 OE commercialisé sous la dénomination TWEEN 80, le mono-oléate de sorbitan oxyéthylène à 5 OE commercialisé sous la dénomination TWEEN 81, le tri-oléate de sorbitan oxyéthylène à 20 OE commercialisé sous la dénomination TWEEN 85.

De préférence, l'acide gras de l'ester de sorbitan oxyalkyléné est un acide gras saturé.

Les esters de sorbitan préférés sont le mono-laurate de sorbitan oxyéthylène à 4 OE, le mono-laurate de sorbitan oxyéthylène à 20 OE, et leurs mélanges. L'ester de sorbitan particulièrement préféré est le mono-laurate de sorbitan oxyéthylène à 4 OE.

Selon l'invention, l'ester d'acide gras et de sorbitan oxyalkyléné est mis en oeuvre dans une composition aqueuse, ou dans une composition hydroalcoolique.

Par "composition aqueuse", on désigne une composition comprenant au moins 50% en poids d'eau.

Par "composition hydroalcoolique", on désigne une composition comprenant un mélange d'eau et d'au moins un alcool et/ou au moins un polyol cosmétiquement acceptable.

La composition hydroalcoolique peut ainsi comprendre au moins un polyol choisi parmi le glycérol, le propylèneglycol, les polyéthylèneglycols et leurs mélanges.

De préférence, la composition hydroalcoolique comprend au moins un alcool inférieur en C₁-C₄, tels que par exemple l'éthanol, l'isopropanol, le tertio-butanol, le n-butanol et leurs mélanges. Encore plus préférentiellement, la composition hydroalcoolique contient de l'éthanol.

On peut également employer dans le cadre de la présente invention un mélange d'alcool(s) et de polyol(s) tels que décrits ci-avant.

Le rapport pondéral entre la quantité d'eau d'une part et la quantité d'alcool(s) et de polyol(s) d'autre part dans la composition hydroalcoolique est de préférence compris entre 0,05 et 20.

De manière plus préférée, ce rapport est compris entre 0,1 et 10, plus préférentiellement entre 0,25 et 4, mieux encore entre 0,5 et 2.

La teneur totale en alcool(s) et polyol(s) de la composition hydroalcoolique est de préférence comprise entre 5 et 95 % en poids, plus préférentiellement entre 10 et 90 % en poids, encore plus préférentiellement entre 20 et 80 % en poids, et mieux encore entre 20 et 70 % en poids.

Selon la présente invention, l'ester d'acide gras et de sorbitan oxyalkyléné est utilisé en tant qu'agent apaisant dans une composition aqueuse, c'est-à-dire une composition comprenant au moins 50% en poids d'eau, ou dans une composition hydroalcoolique, c'est-à-dire une composition comprenant au moins 50% en poids du mélange d'eau et d'alcool(s) et/ou polyol(s) précité.

A cet effet, l'ester d'acide gras et de sorbitan oxyalkyléné peut être incorporé en tant qu'agent apaisant dans toute composition de traitement cosmétique capillaire qui comprend de l'eau et/ou au moins un alcool et/ou au moins un polyol dans les teneurs minimales précitées.

Il peut ainsi par exemple, et de manière non limitative, être incorporé en tant qu'agent apaisant dans des compositions cosmétiques capillaires classiques, telles que des compositions de lavage des cheveux et du cuir chevelu, des compositions de coloration, de décoloration, de permanente ou de défrisage des cheveux, des compositions de mise en forme des cheveux telles que des laques, des gels, des mousses, des compositions de traitement cosmétique du cuir chevelu tels que des traitements antipelliculaires, des traitements antichute, des traitements hydratants, des traitements nourrissants, des traitements apaisants. Il peut également être incorporé dans une composition de prétraitement du cuir chevelu.

Selon un mode de réalisation, la composition cosmétique destinée à apaiser le cuir chevelu se présente sous forme de shampooing ou d'après-shampooing.

La composition des shampooings et des après-shampooings est largement connue de l'homme du métier.

Les shampooings comprennent classiquement une base lavante constituée de un ou plusieurs tensioactif(s) choisi(s) parmi les tensioactifs anioniques, non ioniques, amphotères ou zwittérioniques.

Les après shampooings comprennent classiquement au moins un agent conditionneur tel que par exemple des tensioactifs cationiques, des polymères cationiques, des polymères amphotères ou zwittérioniques, des silicones. Les shampooings peuvent également comprendre de tels agents conditionneurs.

Les shampooings et après shampooings peuvent également contenir, de manière non exhaustive et connue en soi, un ou plusieurs corps gras tels que par exemple les huiles végétales, les huiles animales, les huiles minérales, les huiles naturelles ou synthétiques, les cires, les alcools gras, un ou plusieurs additifs classiques tels que les agents antichute, les agents oxydants, les céramides et pseudo-céramides, les vitamines et pro-vitamines dont le panthénol, les céramides et pseudo-céramides, les filtres solaires, les pigments minéraux ou organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux ou organiques, les agents anti-oxydants, les hydroxyacides, les parfums et les agents conservateurs.

La composition cosmétique destinée à apaiser le cuir chevelu peut également être une composition antipelliculaire, telle que un shampooing antipelliculaire, un après-shampooing antipelliculaire.

Les shampooings et après-shampooings antipelliculaires se caractérisent alors en ce qu'ils contiennent, outre les ingrédients classiques des shampooings et après-shampooings, au moins un ester d'acide gras et de sorbitan oxyalkyléné comprenant de 1 à 20 unités oxyalkylène, et au moins un agent antipelliculaire. Comme agents antipelliculaires, peuvent être employés par exemple des composés tels que la piroctone olamine, la pyrithione de zinc, l'acide salicylique, le disulfure de sélénium, et leurs mélanges.

Les compositions de shampooings et après-shampooings antipelliculaires comprennent classiquement de 0,001 à 10 % en poids d'agent(s) antipelliculaire(s), de préférence de 0,1 à 5 % en poids, encore plus préférentiellement de 0,2 à 2 % en poids, par rapport au poids total de la composition.

Selon un mode de mise en oeuvre préféré, l'ester d'acide gras et de sorbitan oxyalkyléné est incorporé dans une lotion hydroalcooliq.ue de traitement cosmétique du cuir chevelu. Ainsi, la composition cosmétique destinée à apaiser le cuir chevelu est une lotion hydroalcoolique de traitement cosmétique du cuir chevelu.

Par lotion hydroalcoolique, on désigne une lotion contenant au moins 80% en poids, de préférence au moins 90% en poids, et mieux encore au moins 95% en poids du mélange d'eau et d'au moins un alcool et/ou polyol précité.

Il peut s'agir d'une lotion classique, ou d'une lotion antipelliculaire, c'est-à-dire une lotion comprenant au moins un agent antipelliculaire tel que par exemple ceux cités ci-avant.

Une telle lotion antipelliculaire comprend classiquement de 0,001 à 10 % en poids d'agent(s) antipelliculaire(s), de préférence de 0,01 à 5 % en poids, encore plus préférentiellement de 0,02 à 2 % en poids, par rapport au poids total de la composition.

Selon l'invention, la composition cosmétique comprend de préférence au moins 0,01 % en poids d'ester d'acide gras et de sorbitan oxyalkyléné, par rapport au poids total de la composition. De manière plus préférée, ladite composition comprend de 0,05% en poids à 10% en poids d'ester d'acide gras et de sorbitan oxyalkyléné, plus préférentiellement de 0,1 à 8 % en poids, mieux encore de 0,2 à 5% en poids, par rapport au poids total de la composition.

Les exemples suivants sont donnés à titre illustratif de la présente invention, et ne sauraient en limiter la portée.

Dans ces exemples, toutes les quantités sont indiquées en pour cent en poids de matière active par rapport au poids total de la composition, sauf indication contraire.

### EXEMPLES

### Exemple 1 :

Une lotion A, conforme à l'invention, et une lotion B comparative ont été préparées à partir des ingrédients indiqués dans le tableau ci-dessous.

| Composition | Lotion A | Lotion B |
|---|---|---|
| mono-laurate de sorbitan oxyéthylène à 4 OE (Tween 21 de Uniqema) | 0,5 | 0 |
| huile de ricin éthoxylée (Cremophor RH 40 de BASF) | 0,3 | 0,3 |
| éthanol | 40,6 | 40,6 |
| eau qsp | 100% | 100% |

Un test d'efficacité de l'effet apaisant des lotions A et B par rapport à un inducteur irritant (la capsaïcine) a été effectué, de la manière suivante :
les lotions A et B ont été appliquées chacune sur une petite zone du cuir chevelu (les cheveux ayant été rasés sur les zones d'application) de 29 sujets possédant des cuirs chevelus sensibles.

Puis a été appliquée, sur les deux zones d'application de chaque sujet, une crème comprenant 0,025 % en poids de capsaïcine.

L'évaluation des réactions d'inconfort du cuir chevelu (échauffement, démangeaisons, picotement et rougeurs) a été effectuée avant toute application de produit sur le cuir chevelu, puis 10 minutes après la pause de chaque lotion, puis à différents temps de pause de la crème à base de capsaïcine.

Les sensations d'échauffement, de démangeaisons et de picotement ont été chacune évaluées et notées par chaque sujet, sur une échelle croissante (plus la note attribuée est élevée, plus la sensation ressentie est importante). L'apparition de rougeurs a été observé et noté (également sur une échelle croissante), pour chaque sujet, par l'expérimentateur.

Les réactions d'inconfort obtenues avec la lotion A d'une part, et avec la lotion B d'autre part, ont été traités par analyse statistique (moyenne pour chaque réaction d'inconfort, et pour l'inconfort cumulé : échauffement + démangeaisons + picotement + rougeurs).

Les résultats obtenus sont les suivants :
- en ce qui concerne les rougeurs, la valeur moyenne obtenue est de 4,3 pour la lotion A, et 6,7 pour la lotion B, avec une valeur p de 0,005.
- en ce qui concerne l'inconfort cumulé, la valeur moyenne obtenue est de 10 pour la lotion A, et 14,1 pour la lotion B avec une valeur p de 0,020.

Ainsi, l'application sur le cuir chevelu de la lotion A conforme à l'invention s'est avérée présenter un effet apaisant largement supérieur à celui obtenu par application de la lotion comparative B.

En particulier, a été observée une diminution significative des rougeurs, ainsi que de l'inconfort cumulé.

### Exemple 2 :

Cet exemple détaille la formulation d'une composition de prétraitement du cuir chevelu, destinée à être appliquée avant un traitement de coloration ou de défrisage des cheveux.

| Composition | Teneur |
|---|---|
| mélange d'alcool cétylstéarylique (80 % en poids) et d'alcool cétylstéarylique oxyéthyléné à 30 OE (20 % en poids) (Sinnowax AO de Cognis) | 7,5 g |
| mono-laurate de sorbitan oxyéthylène à 4 OE (Tween 21 de Uniqema) | 4g |
| conservateurs | qs |
| eau | qsp 100g |

Appliquée sur le cuir chevelu avant un traitement de coloration ou de défrisage des cheveux, cette composition permet de prévenir de manière efficace l'apparition des réactions d'inconfort susceptibles de survenir à la suite du traitement de coloration ou de défrisage.

## Revendications

1. Ester d'acide gras et de sorbitan oxyalkyléné comprenant de 1 à 20 unités oxyalkylène, pour son utilisation en tant qu'agent apaisant dans une composition cosmétique aqueuse ou hydroalcoolique destinée au soin du cuir chevelu.

2. Ester d'acide gras et de sorbitan selon la revendication 1, **caractérisé en ce qu'**il est oxyéthyléné.

3. Ester d'acide gras et de sorbitan selon la revendication précédente, **caractérisé en ce qu'**il est choisi parmi les dérivés oxyéthylénés des mono et des polyesters d'acides gras en C₈₋₃₀ et du sorbitan, ayant de 1 à 20 motifs d'oxyde d'éthylène.

4. Ester d'acide gras et de sorbitan selon la revendication précédente, **caractérisé en ce qu'**il est choisi parmi les dérivés oxyéthylénés des mono et des polyesters d'acides gras en C₁₂₋₂₄ et du sorbitan, ayant de 4 à 20 motifs d'oxyde d'éthylène.

5. Ester d'acide gras et de sorbitan selon la revendication précédente, **caractérisé en ce qu'**il est choisi parmi le monolaurate de sorbitan oxyéthylène à 4 OE, le mono-laurate de sorbitan oxyéthylène à 20 OE, et leurs mélanges.

6. Ester d'acide gras et de sorbitan selon la revendication précédente, **caractérisé en ce qu'**il est le mono-laurate de sorbitan oxyéthylène à 4 OE.

7. Ester d'acide gras et de sorbitan selon l'une quelconque des revendications précédentes, **caractérisé en ce que** la composition cosmétique est une composition aqueuse

8. Ester d'acide gras et de sorbitan selon l'une quelconque des revendications 1 à 6, **caractérisé en ce que** la composition cosmétique est une composition hydroalcoolique comprenant un mélange d'eau et d'au moins un alcool et/ou au moins un polyol cosmétiquement acceptable.

9. Ester d'acide gras et de sorbitan selon la revendication précédente, **caractérisé en ce que** la composition hydroalcoolique comprend au moins un polyol choisi parmi le glycérol, le propylèneglycol, les polyéthylèneglycols et leurs mélanges.

10. Ester d'acide gras et de sorbitan selon l'une quelconque des revendications 8 et 9, **caractérisé en ce que** la composition hydroalcoolique comprend au moins un alcool inférieur en C₁-C₄, tel que par exemple l'éthanol, l'isopropanol, le tertio-butanol, le n-butanol et leurs mélanges.

11. selon la revendication précédente, **caractérisé en ce que** la composition hydroalcoolique contient de l'éthanol.

12. Ester d'acide gras et de sorbitan selon l'une quelconque des revendications 8 à 11, **caractérisé en ce que**, dans la composition hydroalcoolique, le rapport pondéral entre la quantité d'eau d'une part et la quantité d'alcool(s) et de polyol(s) d'autre part est compris entre 0,05 et 20.

13. Ester d'acide gras et de sorbitan selon la revendication précédente, **caractérisé e**n ce que le rapport pondéral entre la quantité d'eau d'une part et la quantité d'alcool(s) et de polyol(s) d'autre part est compris entre 0,1 et 10, de préférence entre 0,25 et 4.

14. Ester d'acide gras et de sorbitan selon l'une quelconque des revendications 8 à 13, **caractérisé en ce que** la teneur totale en alcool(s) et polyol(s) est comprise entre 5 et 95 % en poids, plus préférentiellement entre 10 et 90% en poids, encore plus préférentiellement entre 20 et 80 % en poids, par rapport au poids total de la composition hydroalcoolique.

15. Ester d'acide gras et de sorbitan selon l'une quelconque des revendications précédentes, **caractérisé en ce que** la composition cosmétique destinée à apaiser le cuir chevelu se présente sous forme de shampooing ou d'après-shampooing.

16. Ester d'acide gras et de sorbitan selon la revendication précédente, **caractérisé en ce que** la composition cosmétique est un shampooing antipelliculaire ou un après-shampooing antipelliculaire contenant, outre ledit ester d'acide gras et de sorbitan oxyalkyléné ainsi que les ingrédients classiques des shampooings et après-shampooings, au moins un agent antipelliculaire.

17. Ester d'acide gras et de sorbitan selon l'une quelconque des revendications 1 à 14, **caractérisé en ce que** la composition cosmétique destinée à apaiser le cuir chevelu est une lotion hydroalcoolique de traitement cosmétique du cuir chevelu.

18. Ester d'acide gras et de sorbitan selon la revendication précédente, **caractérisé en ce** la lotion hydroalcoolique, contient au moins 80% en poids, de préférence au moins 90% en poids, et mieux encore au moins 95% en poids de mélange d'eau et d'alcool(s) et/ou de polyol(s).

19. Ester d'acide gras et de sorbitan selon l'une quelconque des revendications 17 et 18, **caractérisé en ce que** la composition cosmétique destinée à apaiser le cuir chevelu est une lotion antipelliculaire, contenant au moins un agent antipelliculaire.

20. Ester d'acide gras et de sorbitan selon l'une quelconque des revendications 16 ou 19, **caractérisé en ce que** l'agent antipelliculaire est choisi parmi la piroctone olamine, la pyrithione de zinc, l'acide salicylique, le disulfure de sélénium, et leurs mélanges.

21. Ester d'acide gras et de sorbitan selon l'une quelconque des revendications 16, 19 ou 20, **caractérisé en ce que** le shampooing, l'après-shampooing ou la lotion antipelliculaire comprend de 0,001 à 10 % en poids d'agent(s) antipelliculaire(s), par rapport au poids total de la composition.

22. Ester d'acide gras et de sorbitan selon l'une quelconque des revendications précédentes, **caractérisé en ce que** la composition cosmétique comprend au moins 0,01 % en poids d'ester d'acide gras et de sorbitan oxyalkyléné comprenant de 1 à 20 unités oxyalkylène, par rapport au poids total de la composition.

23. Ester d'acide gras et de sorbitan selon la revendication précédente, **caractérisé en ce que** la composition cosmétique comprend de 0,05% à 10% en poids d'ester d'acide gras et de sorbitan oxyalkyléné comprenant de 1 à 20 unités oxyalkylène, de préférence de 0,1 à 8 % en poids, mieux encore de 0,2 à 5% en poids, par rapport au poids total de la composition.

24. Ester d'acide gras et de sorbitan oxyalkyléné comprenant de 1 à 20 unités oxyalkylène pour son utilisation pour diminuer l'intensité des réactions d'inconfort du cuir chevelu, voire de les faire disparaître.

25. Procédé de traitement cosmétique capillaire comprenant au moins deux étapes :
- une première étape de prétraitement comprenant l'application sur le cuir chevelu d'une composition cosmétique comprenant au moins un ester d'acide gras et de sorbitan oxyalkyléné comprenant de 1 à 20 unités oxyalkylène tel que défini dans l'une quelconque des revendications 1 à 6, et
- une deuxième étape, postérieure à ladite première étape, de traitement cosmétique capillaire choisi parmi une coloration, une décoloration, une permanente, un défrisage.

## Claims

1. Oxyalkylenated ester of fatty acid and sorbitan comprising from 1 to 20 oxyalkylene units for use as a soothing agent in an aqueous or aqueous-alcoholic cosmetic composition intended for care of the scalp.

2. Ester of fatty acid and sorbitan according to Claim 1, **characterized in that** it is oxyethylenated.

3. Ester of fatty acid and sorbitan according to the preceding claim, **characterized in that** it is selected from the oxyethylenated derivatives of mono and polyesters of C₈₋₃₀ fatty acids and sorbitan, having from 1 to 20 ethylene oxide units.

4. Ester of fatty acid and sorbitan according to the preceding claim, **characterized in that** it is selected from the oxyethylenated derivatives of mono and polyesters of C₁₂₋₂₄ fatty acids and sorbitan, having from 4 to 20 ethylene oxide units.

5. Ester of fatty acid and sorbitan according to the preceding claim, **characterized in that** it is selected from oxyethylenated sorbitan monolaurate with 4 OE, oxyethylenated sorbitan monolaurate with 20 OE, and mixtures thereof.

6. Ester of fatty acid and sorbitan according to the preceding claim, **characterized in that** it is oxyethylenated sorbitan monolaurate with 4 OE.

7. Ester of fatty acid and sorbitan according to any one of the preceding claims, **characterized in that** the cosmetic composition is an aqueous composition.

8. Ester of fatty acid and sorbitan according to any one of Claims 1 to 6, **characterized in that** the cosmetic composition is an aqueous-alcoholic composition comprising a mixture of water and at least one cosmetically acceptable alcohol and/or at least one cosmetically acceptable polyol.

9. Ester of fatty acid and sorbitan according to the preceding claim, **characterized in that** the aqueous-alcoholic composition comprises at least one polyol selected from glycerol, propylene glycol, polyethylene glycols and mixtures thereof.

10. Ester of fatty acid and sorbitan according to either one of Claims 8 and 9, **characterized in that** the aqueous-alcoholic composition comprises at least one lower C₁₋C₄ alcohol, for example ethanol, isopropanol, tert-butanol, n-butanol and mixtures thereof.

11. Ester of fatty acid and sorbitan according to the preceding claim, **characterized in that** the aqueous-alcoholic composition contains ethanol.

12. Ester of fatty acid and sorbitan according to any one of Claims 8 to 11, **characterized in that**, in the aqueous-alcoholic composition, the weight ratio of the amount of water on the one hand and the amount of alcohol(s) and polyol(s) on the other hand is between 0.05 and 20.

13. Ester of fatty acid and sorbitan according to the preceding claim, **characterized in that** the weight ratio of the amount of water on the one hand and the amount of alcohol(s) and polyol(s) on the other hand is between 0.1 and 10, preferably between 0.25 and 4.

14. Ester of fatty acid and sorbitan according to any one of Claims 8 to 13, **characterized in that** the total content of alcohol(s) and polyol(s) is between 5 and 95 wt.%, more preferably between 10 and 90 wt.%, and even more preferably between 20 and 80 wt.%, relative to the total weight of the aqueous-alcoholic composition.

15. Ester of fatty acid and sorbitan according to any one of the preceding claims, **characterized in that** the cosmetic composition intended for soothing the scalp is in the form of a shampoo or after-shampoo.

16. Ester of fatty acid and sorbitan according to the preceding claim, **characterized in that** the cosmetic composition is an anti-dandruff shampoo or an anti-dandruff after-shampoo containing, in addition to said oxyalkylenated ester of fatty acid and sorbitan and the conventional ingredients of shampoos and after-shampoos, at least one anti-dandruff agent.

17. Ester of fatty acid and sorbitan according to any one of Claims 1 to 14, **characterized in that** the cosmetic composition intended for soothing the scalp is an aqueous-alcoholic lotion for cosmetic treatment of the scalp.

18. Ester of fatty acid and sorbitan according to the preceding claim, **characterized in that** the aqueous-alcoholic lotion contains at least 80 wt.%, preferably at least 90 wt.%, and better still at least 95 wt.% of a mixture of water and alcohol(s) and/or polyol(s).

19. Ester of fatty acid and sorbitan according to either one of Claims 17 and 18, **characterized in that** the cosmetic composition intended for soothing the scalp is an anti-dandruff lotion containing at least one anti-dandruff agent.

20. Ester of fatty acid and sorbitan according to either one of Claims 16 or 19, **characterized in that** the anti-dandruff agent is selected from piroctone olamine, zinc pyrithione, salicylic acid, selenium disulphide and mixtures thereof.

21. Ester of fatty acid and sorbitan according to any one of Claims 16, 19 or 20, **characterized in that** the shampoo, the after-shampoo or the anti-dandruff lotion contains from 0.001 to 10 wt.% of anti-dandruff agent(s), relative to the total weight of the composition.

22. Ester of fatty acid and sorbitan according to any one of the preceding claims, **characterized in that** the cosmetic composition contains at least 0.01 wt.% of oxyalkylenated ester of fatty acid and sorbitan comprising from 1 to 20 oxyalkylene units, relative to the total weight of the composition.

23. Ester of fatty acid and sorbitan according to the preceding claim, **characterized in that** the cosmetic composition contains from 0.05% to 10 wt.% of oxyalkylenated ester of fatty acid and sorbitan comprising from 1 to 20 oxyalkylene units, preferably from 0.1 to 8 wt.%, better still from 0.2 to 5 wt.%, relative to the total weight of the composition.

24. Oxyalkylenated ester of fatty acid and sorbitan comprising from 1 to 20 oxyalkylene units for use in reducing the intensity of the reactions of discomfort of the scalp, or even making them disappear.

25. Method of cosmetic hair treatment comprising at least two steps:
- a first pretreatment step comprising the application, on the scalp, of a cosmetic composition comprising at least one oxyalkylenated ester of fatty acid and sorbitan comprising from 1 to 20 oxyalkylene units as defined in any one of Claims 1 to 6, and
- a second step, subsequent to the said first step, of cosmetic hair treatment selected from a colouring, bleaching, permanent-waving and hair-straightening treatment.

## Patentansprüche

1. Oxyalkylenierter Sorbitanfettsäureester mit 1 bis 20 Oxyalkylen-Einheiten zur Verwendung als beruhigendes Mittel in einer wäßrigen oder wäßrigalkoholischen kosmetischen Zusammensetzung zur Pflege der Kopfhaut.

2. Sorbitanfettsäureester nach Anspruch 1, **dadurch gekennzeichnet, daß** er oxyethyleniert ist.

3. Sorbitanfettsäureester nach dem vorhergehenden Anspruch, **dadurch gekennzeichnet, daß** er unter oxyethylenierten Derivaten von Sorbitanmono-C₈₋₃₀-fettsäureestern und Sorbitanpoly-C₈₋₃₀-fettsäureestern mit 1 bis 20 Ethylenoxid-Einheiten ausgewählt ist.

4. Sorbitanfettsäureester nach dem vorhergehenden Anspruch, **dadurch gekennzeichnet, daß** er unter oxyethylenierten Derivaten von Sorbitanmono-C₁₂₋₂₄-fettsäureestern und Sorbitanpoly-C₁₂₋₂₄-fettsäureestern mit 4 bis 20 Ethylenoxid-Einheiten ausgewählt ist.

5. Sorbitanfettsäureester nach dem vorhergehenden Anspruch, **dadurch gekennzeichnet, daß** er unter oxyethyleniertem Sorbitanmonolaurat mit 4 EO, oxyethyleniertem Sorbitanmonolaurat mit 20 EO und Mischungen davon ausgewählt ist.

6. Sorbitanfettsäureester nach dem vorhergehenden Anspruch, **dadurch gekennzeichnet, daß** es sich um oxyethyleniertes Sorbitanmonolaurat mit 4 EO handelt.

7. Sorbitanfettsäureester nach einem der vorhergehenden Ansprüche, **dadurch gekennzeichnet, daß** es sich bei der kosmetischen Zusammensetzung um eine wäßrige Zusammensetzung handelt.

8. Sorbitanfettsäureester nach einem der Ansprüche 1 bis 6, **dadurch gekennzeichnet, daß** es sich bei der kosmetischen Zusammensetzung um eine wäßrig-alkoholische Zusammensetzung, die eine Mischung von Wasser und mindestens einem Alkohol und/oder mindstens einem kosmetisch unbedenklichen Polyol umfaßt, handelt.

9. Sorbitanfettsäureester nach dem vorhergehenden Anspruch, **dadurch gekennzeichnet, daß** die wäßrig-alkoholische Zusammensetzung mindestens ein unter Glycerin, Propylenglykol, Polyethylenglykolen und Mischungen davon ausgewähltes Polyol umfaßt.

10. Sorbitanfettsäureester nach einem der Ansprüche 8 und 9, **dadurch gekennzeichnet, daß** die wäßrig-alkoholische Zusammensetzung mindestens einen niederen C₁-C₄-Alkohol, wie beispielsweise Ethanol, Isopropanol, tert.-Butanol, n-Butanol und Mischungen davon, umfaßt.

11. Sorbitanfettsäureester nach dem vorhergehenden Anspruch, **dadurch gekennzeichnet, daß** die wäßrig-alkoholische Zusammensetzung Ethanol enthält.

12. Sorbitanfettsäureester nach einem der Ansprüche 8 bis 11, **dadurch gekennzeichnet, daß** in der wäßrigalkoholischen Zusammensetzung das Gewichtsverhältnis zwischen der Menge an Wasser einerseits und der Menge an Alkohol(en) und Polyol(en) andererseits zwischen 0,05 und 20 liegt.

13. Sorbitanfettsäureester nach dem vorhergehenden Anspruch, **dadurch gekennzeichnet, daß** das Gewichtsverhältnis zwischen der Menge an Wasser einerseits und der Menge an Alkohol(en) und Polyol(en) andererseits zwischen 0,1 und 10, vorzugsweise zwischen 0,25 und 4, liegt.

14. Sorbitanfettsäureester nach einem der Ansprüche 8 bis 13, **dadurch gekennzeichnet, daß** der Gesamtgehalt an Alkohol(en) und Polyol(en) zwischen 5 und 95 Gew.-%, weiter bevorzugt zwischen 10 und 90 Gew.-%, noch weiter bevorzugt zwischen 20 und 80 Gew.-%, bezogen auf das Gesamtgewicht der wäßrigalkoholischen Zusammensetzung, liegt.

15. Sorbitanfettsäureester nach einem der vorhergehenden Ansprüche, **dadurch gekennzeichnet, daß** die kosmetische Zusammensetzung zur Beruhigung der Kopfhaut in Form eines Shampoos oder einer Haarpflegespülung vorliegt.

16. Sorbitanfettsäureester nach dem vorhergehenden Anspruch, **dadurch gekennzeichnet, daß** es sich bei der kosmetischen Zusammensetzung um ein Antischuppen-Shampoo oder eine Antischuppen-Haarpflegespülung, das neben dem oxyalkylenierten Sorbitanfettsäureester und den herkömmlichen Bestandteilen von Shampoos und Haarpflegespülungen mindestens ein Antischuppenmittel enthält, handelt.

17. Sorbitanfettsäureester nach einem der Ansprüche 1 bis 14, **dadurch gekennzeichnet, daß** es sich bei der kosmetischen Zusammensetzung zur Beruhigung der Kopfhaut um eine wäßrig-alkoholische Lotion zur kosmetischen Behandlung der Kopfhaut handelt.

18. Sorbitanfettsäureester nach dem vorhergehenden Anspruch, **dadurch gekennzeichnet, daß** die wäßrig-alkoholische Lotion mindestens 80 Gew.-%, vorzugsweise mindestens 90 Gew.-% und noch besser 95 Gew.-% einer Mischung von Wasser und Alkohol(en) und/oder Polyol(en) enthält.

19. Sorbitanfettsäureester nach einem der Ansprüche 17 und 18, **dadurch gekennzeichnet, daß** es sich bei der kosmetischen Zusammensetzung zur Beruhigung der Kopfhaut um eine Antischuppen-Lotion, die mindestens ein Antischuppenmittel enthält, handelt.

20. Sorbitanfettsäureester nach einem der Ansprüche 16 oder 19, **dadurch gekennzeichnet, daß** das Schuppenmittel unter Piroctonolamin, Zinkpyrithion, Salicylsäure, Selendisdulfid und Mischungen davon ausgewählt ist.

21. Sorbitanfettsäureester nach einem der Ansprüche 16, 19 oder 20, **dadurch gekennzeichnet, daß** das Shampoo, die Haarpflegespülung bzw. die Antischuppen-Lotion 0,001 bis 10 Gew.-% eines oder mehrerer Antischuppenmittel, bezogen auf das Gesamtgewicht der Zusammensetzung, umfaßt.

22. Sorbitanfettsäureester nach einem der vorhergehenden Ansprüche, **dadurch gekennzeichnet, daß** die kosmetische Zusammensetzung mindestens 0,01 Gew.-% oxyalkylenierten Sorbitanfettsäureester mit 1 bis 20 Oxyalkylen-Einheiten, bezogen auf das Gesamtgewicht der Zusammensetzung, umfaßt.

23. Sorbitanfettsäureester nach dem vorhergehenden Anspruch, **dadurch gekennzeichnet, daß** die kosmetische Zusammensetzung 0,05 bis 10 Gew.-% oxyalkylenierten Sorbitanfettsäureester mit 1 bis 20 Oxyalkylen-Einheiten, vorzugsweise 0,1 bis 8 Gew.-%, noch besser 0,2 bis 5 Gew.-%, bezogen auf das Gesamtgewicht der Zusammensetzung, umfaßt.

24. Oxyalkylenierter Sorbitanfettsäureester mit 1 bis 20 Oxyalkylen-Einheiten zur Verwendung zur Verringerung der Intensität oder Beseitigung von Unbehaglichkeitsreaktionen der Kopfhaut.

25. Verfahren zur kosmetischen Haarbehandlung mit mindestens zwei Schritten:
- einem ersten Vorbehandlungsschritt, bei dem man eine kosmetische Zusammensetzung, die mindestens einen oxyalkylenierten Sorbitanfettsäureester mit 1 bis 20 Oxyalkylen-Einheiten gemäß einem der Ansprüche 1 bis 6 umfaßt, auf die Kopfhaut aufbringt, und
- einem auf den ersten Schritt folgenden zweiten Schritt von kosmetischen Haarbehandlung, ausgewählt unter einer Färbung, einer Entfärbung, einer Dauerwelle und einer Entkrausung.
